(19) Europäisches Patentamt
European Patent Office
Office européen des brevets

(11) Veröffentlichungsnummer: 0 423 837 A2

(12) **EUROPÄISCHE PATENTANMELDUNG**

(21) Anmeldenummer: **90121087.2**

(51) Int. Cl.5: **C07D 501/46**

(22) Anmeldetag: **04.04.85**

Diese Anmeldung is am 03 - 11 - 1990 als Teilanmeldung zu der unter INID-Kode 60 erwähnten Anmeldung eingereicht worden.

(30) Priorität: **10.04.84 AT 1198/84**

(43) Veröffentlichungstag der Anmeldung: **24.04.91 Patentblatt 91/17**

(60) Veröffentlichungsnummer der früheren Anmeldung nach Art. 76 EPÜ: **0 185 679**

(84) Benannte Vertragsstaaten: **BE CH DE FR GB IT LI**

(71) Anmelder: **BIOCHEMIE GESELLSCHAFT M.B.H.**

**A-6250 Kundl(AT)**

(72) Erfinder: **Ascher, Gerd**

**A-6250 Kundl(AT)**
Erfinder: **Sturm, Herbert**
**Urichstrasse 102**
**A-6500 Landeck(AT)**
Erfinder: **Thaler, Heinrich**

**verstorben(AT)**
Erfinder: **Veit, Werner**
**Kaiserjägerstrasse 27**
**A-6330 Kufstein(AT)**

(74) Vertreter: **Kleine Deters, Johannes et al**
**c/o Sandoz Technology LTD.,**
**Patantabteilung**
**CH-4002 Basel(CH)**

(54) **Cephalosporinzwischenprodukte und ihre Verwendung.**

(57) 1. Cephalosporinzwischenprodukte der Formel

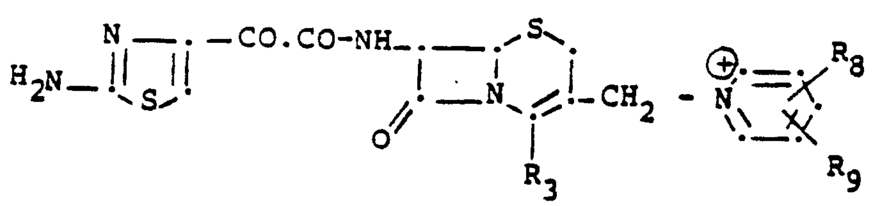

I

worin $R_3$ die Carboxyl-, die Carboxylat- oder eine Carbonsäureestergruppe bedeutet und $R_8$ und $R_9$ gleich oder verschieden sind und jeweils für Wasserstoff, Halogen, Alkyl, Hydroxy, Carboxamido, Alkoxycarbonyl, Amino, Monoalkylamino oder Dialkylamino stehen oder zusammen einen gegebenenfalls substituierten, 5- oder 6-gliedrigen carbocylischen Ring bedeuten und ihre Verwendung.

EP 0 423 837 A2

# CEPHALOSPORINZWISCHENPRODUKTE UND IHRE VERWENDUNG

Die Erfindung betrifft Cephalosporinzwischenprodukte der Formel

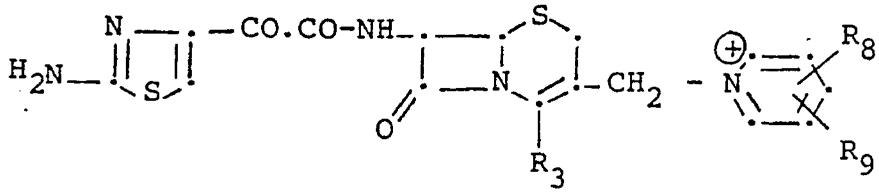

I

wobei $R_8$ und $R_9$ gleich oder verschieden sind und jeweils für Wasserstoff, Halogen, Alkyl, Hydroxy, Carboxamido, Alkoxycarbonyl, Amino, Monoalkylamino oder Dialkylamino stehen oder zusammen einen gegebenenfalls substituierten, 5- oder 6-gliedrigen carbocyclischen Ring bedeuten, steht und $R_3$ die Carboxyl-, die Carboxylat- oder eine Carbonsäureestergruppe bedeutet, und ihre Verwendung.

Aus den Cephalosporinzwischenprodukten der Formel I können wertvolle Cephalosporinantibiotika hergestellt werden.

Wie auf dem Gebiet der Cephalosporine bekannt, können die Verbindungen in Form der freien Säuren ($R_3$ = COOH) oder von Salzen, beispielsweise von Alkali- oder Erdalkalimetallsalzen, vorzugsweise von Alkalimetallsalzen, wie Natriumsalzen, vorliegen. Weiters können die Verbindungen auch in Form von Estern, beispielsweise in Form des Pivaloyloxymethylesters vorliegen, aber auch als andere Ester, wie beispielsweise Acetoxymethyl-, 1-Acetoxyethyl-, 1-Ethoxycarbonyloxyethyl-, 5-Indanoyl- oder vorzugsweise Hexanoylmethyl-, Phthalidyl-, Carbethoxymethoxymethyl- oder 3-Carbethoxy-1-acetonylester. Die Verbindungen der Formel I können auch als innere Salze vorliegen.

Wie bereits erwähnt, sind die aus den Verbindungen der Formel I herstellbaren Cephalosporinantibiotika bekannt, und verschiedene Methoden zu ihrer Herstellung wurden bereits vorgeschlagen. Eine dieser Methoden beinhaltet die Acylierung des entsprechenden, gegebenenfalls geschützten 7-Aminocephalosporansäurederivats mit einem aktiven Derivat einer Säure der Formel

$R_1$ - COOH    A

worin $R_1$ für eine Gruppe der Formel

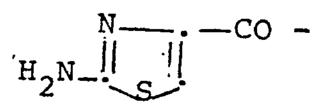

II

steht.

Die verschiedenen vorgeschlagenen aktiven Derivate beinhalten auch aktive Ester.

Eine bei der Herstellung der Verbindungen der Formel I entstehende Schwierigkeit besteht darin, daß in der Praxis der Aminosubstituent im Thiazolylring der Seitenkette vor der Aktivierung der Säurefunktion geschützt werden muß, da sonst kompetitive Reaktionen zu starken Ausbeuteverminderungen an Endprodukt führen können. Die Einführung von Schutzgruppen vor dem Acylierungsschritt und ihre spätere Abspaltung verursacht aber meist verminderte Ausbeuten und Reinheit des Endproduk- tes und erhöht wesentlich Reaktionszeit, Energie, Aufwand und Kosten.

Insbesondere umfasst EP 8500156 ein Verfahren zur Herstellung von Verbindungen der Formel I und ihrer Salze, das dadurch gekennzeichnet ist, dass man eine Verbindung der Formel

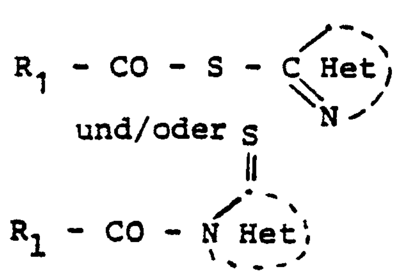

IV

und/oder

IVa

worin $R_1$ obige Bedeutung besitzt, und

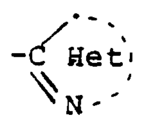

bzw.

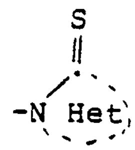

für einen 5- oder 6-gliedrigen Heterocyclus, der zusätzlich zum N-Atom ein oder zwei weitere Heteroatome beinhalten kann, beispielsweise O, N und S, und der substituiert oder mit einem gegebenenfalls substituierten Benzolring annelliert sein kann, stehen, mit einer Verbindung der Formel

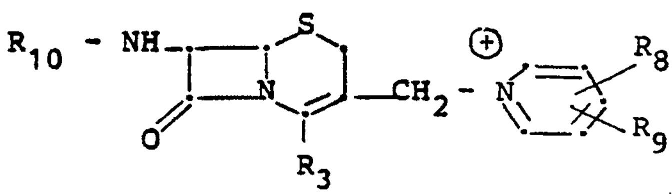

V

worin $R_3$,$R_8$ und $R_9$ obige Bedeutung besitzen und $R_{10}$ für Wasserstoff oder eine Aminoschutzgruppe steht, umsetzt und gewünschtenfalls ein erhaltenes Endprodukt entschützt und gegebenenfalls ein erhaltenes Endprodukt, worin $R_3$ für COOH steht, in ein Salz überführt oder umgekehrt.

Die Verbindungen der Formel IV können auch in Form der Formel IVa vorliegen, wobei das Verhältnis der beiden Formen zueinander von den Reaktionspartnern und den Reaktionsbedingungen abhängig ist. Wenn Verbindungen der Struktur der Formel IV angeführt werden, so umfassen diese immer auch Verbindungen der Struktur der Formel IVa.

Das Verfahren wird vorzugsweise in einem inerten Lösungsmittel oder in einem Gemisch eines solchen Lösungsmittels mit Wasser, z.B. in einem chlorierten Kohlenwasserstoff, wie Dichlormethan, oder in einem Säureester, wie Essigsäureethylester, oder in Azeton, Dimethylformamid oder Dimethylsulfoxid bei einer Temperatur von -40 bis +60° C, insbesondere bei -15 bis +25° C und vorteilhafterweise bei 0 bis 20° C ausgeführt. Die Reaktionsdauer beträgt etwa 1/2 bis 48 Stunden. Die Reaktanden der Formel IV und V werden entweder in stöchiometrischen Mengen eingesetzt oder es wird ein Überschuß von bis zu 25 % an Verbindung der Formel IV verwendet.

Bei der Herstellung von Verbindungen der Formel I, worin $R_3$ COOH bedeutet, wie auch ihrer Salze kann die Carboxylgruppe im Ausgangsprodukt der Formel V geschützt sein. Geeignete Schutzgruppen sind bekannt und umfassen nicht nur die oben angeführten Bedeutungen, sondern auch Silylesterschutzgruppen, insbesondere die Trimethylsilylschutzgruppe, die z.B durch Reaktion der freien Säure mit N,O-Bis-(trimethylsilyl)acetamid eingeführt werden kann.

Die 7-Aminogruppe des Ausgangsmaterials der Formel V kann ebenfalls geschützt sein. Geeignete Schutzgruppen sind bekannt und umfassen z.B. die Trimethylsilylgruppe, die beispielsweise gleichzeitig mit Schützung der Carboxylgruppe eingeführt werden kann.

Der Aminosubstituent im Ausgangsmaterial IV/IVa kann in freier oder geschuetzter Form vorliegen. Wie bereits ausgeführt, ist im allgemeinen ein Schutz nicht notwendig. Sollte jedoch trotzdem ein Schutz erwünscht sein, so kann dies in bekannter Weise, unter Verwendung geeigneter bekannter Schutzgruppen, durchgeführt werden.

Nach der Reaktion der Verbindungen der Formel IV und V können nachfolgende Entschützungsreaktionen in bekannter Weise durchgeführt werden. Ebenso kann die Überführung der freien Säureform ($R_3$ = COOH) in die Salze nach an sich bekannten Methoden ausgeführt werden.

Die Endprodukte können nach bekannten Methoden isoliert und gereinigt werden.

Im obigen Verfahren verwendet man als reaktives Derivat der Säure der Formel A heterocyclische Thioester. Es wurde gefunden, dass eine im Heterocyclus dieser Ester vorhandene Aminogruppe nicht zur Selbstreaktion führt. Demzufolge ist ein Schützen dieser Aminogruppe in der folgenden Acylierung nicht notwendig. Andererseits kann die Aminogruppe selbstverständlich geschützt werden, falls dies aus einem anderen Grund gewünscht wird.

Bei den Verbindungen der Formel IV ist die Art des Ringes

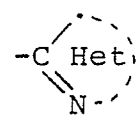

bzw.

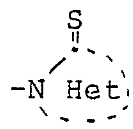

nicht kritisch, die bevorzugten Verbindungen werden durch Faktoren wie Leichtigkeit bei der Herstellung und Verfügbarkeit des Ausgangsmaterials bestimmt. Vorzugsweise bedeutet der Ring 2-Pyridyl oder insbesondere 2-Benzothiazolyl. Er kann auch für Pyrimidinyl, Triazolyl oder Thiazolyl stehen.

Die Verbindungen der Formel IV können durch Veresterung von Verbindungen der Formel

$R_1$ - COOH VI

worin $R_1$ obige Bedeutung besitzt, erhalten werden.

Die Veresterung kann beispielsweise durch Reaktion mit einer Verbindung der Formel

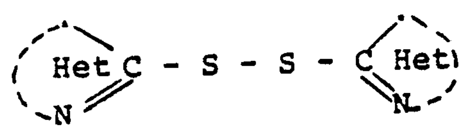 VII

worin beide

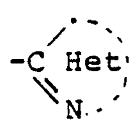

gleich sind und obige Bedeutung besitzen, durchgeführt werden.

Diese Veresterung wird vorzugsweise unter Gegenwart eines Tri(niederalkyl)-oder Tri(aryl)phosphins oder -phosphits, insbesondere Triphenylphosphins, durchgeführt, und zwar vorzugsweise bei einer Temperatur von -30 bis +50° C, insbesondere bei -20 bis +25° C und vorteilhafterweise bei -5 bis +5° C. Als Lösungsmittel wird ein inertes, nicht hydroxylgruppenhältiges organisches Lösungsmittel, z.B. ein chlorierter Kohlenwasserstoff, wie Dichlormethan, eingesetzt. Wird eine Verbindung der Formel IV gewünscht, worin die Aminogruppe in $R_1$ geschützt ist, so kann die Aminoschutzgruppe vor oder nach der Veresterung eingeführt werden. Die Endprodukte können durch Behandeln mit niederen Alkoholen kristallisiert und gereinigt werden, wobei darauf geachtet werden muss, dass die Temperatur 20° C, vorzugsweise 0° C,nicht übersteigt.

Die Verbindungen der Formel I sind, wie bereits erwähnt, Zwischenprodukte zur Herstellung bekannter Antibiotika. Diese entfalten eine Hemmwirkung gegen Bakterien, wie sich durch Untersuchungen in vitro mit dem Reihenverdünnungstest und in vivo durch Versuche an Mäusen, unter Verwendung verschiedener Stämme, z.B. von Staphylococcus aureus, Streptococcus pyogenes, Streptococcus faecalis, Escherichia coli, Proteus vulgaris, Proteus mirabilis, Proteus morganii, Shigella dysenteria, Shigella sonnei, Shigella flexneri, Alcaligenes faecalis, Klebsiella aerogenes, Klebsiella pneunomiae, Serrata marcescens, Salmonella Heidelberg, Salmonella typhimurium, Salmonella enteritidis und Neisseria gonorrhoae, zeigen läßt. Diese Hemmwirkung wurde ab einer Konzentration von ca. 0,01 bis 50 µg/ml bzw. ab einer Dosis von ca. 0,1 bis 100 mg/kg Körpergewicht festgestellt.

Daher können diese Cephalosporinantibiotika als antibakteriell wirksame Antibiotika verwendet werden. Für diese Verwendung hängt die zu verabreichende Dosis von der verwendeten Verbindung und der Verabreichungsart sowie der Behandlungsart ab. Man erhält zufriedenstellende Ergebnisse bei Verabreichung einer täglichen Dosis von ca. 1 bis 6 g. Diese Menge kann gegebenenfalls in entsprechend kleineren

Dosen von 0,25 bis 3 g zwei- bis viermal täglich oder in Retardform gegeben werden.

Die Verbindungen, worin $R_3$ für Carboxyl steht, können in Form der freien Säuren oder ihrer physiologisch verträglichen Salze eingesetzt werden, wobei die Salze größenordnungsmäßig die gleiche Wirksamkeit besitzen wie die freien Säuren. Geeignete Salzformen sind beispielsweise Alkali- und Erdalkalimetallsalze, vorzugsweise Alkalimetallsalze, insbesondere Natriumsalze. Die Verbindungen können gemeinsam mit anorganischen oder organischen pharmakologisch indifferenten Verdünnungs- oder Trägerstoffen bzw. Hilfsstoffen verabreicht werden. Beispielsweise werden sie als Bestandteil von Kapseln, Injektions- oder Instillationszubereitungen eingesetzt.

In den nachfolgenden Beispielen, die die Erfindung näher erläutern, ihren Umfang aber in keiner Weise einschränken sollen, erfolgen alle Temperaturangaben in Celsiusgraden.

Beispiel 1 : 7-[[(2-Amino-4-thiazolyl)-2-oxoacetyl]amino]-3-(1-pyridinium)methyl-8-oxo-5-thia-1-azabicyclo-[4.2.0]oct-2-en-2-carbonsäure.Chlorid:

3,28 g 7-Amino-3-(1-pyridiniummethyl)ceph-3-em-4-carbonsäure.Chlorid werden in 25 ml Dichlormethan suspendiert und mit 3 ml N,O-Bis-(trimethylsilyl)acetamid versetzt. Nach 10 Minuten liegt eine klare Lösung vor. Man kühlt auf -15° und gibt 3,9 g 2-(2-Aminothiazol-4-yl)-2-oxothioessigsäure-S-benzothiazol-2-ylester zu. Nach vierstündigem Rühren bei -15° wird der Ansatz in 100 ml Ethanol eingerührt, wobei das Endprodukt ausfällt. Man rührt eine Stunde bei 0°, filtriert, wäscht mit Ethanol und trocknet im Vakuum. Ausbeute: 4,0 g, d.i. 83,0 %. Fp: ab 162° (Zers.).
IR: NH 3300 (breit), 1765, C=O (β-Lactam), 1645, C=O (Amid), 1610, (Amid II).

Beispiel 2 : 7-[[(2-Amino-4-thiazolyl)-2-oxoacetyl]amino]-3-[(2,3-cyclopenteno-1-pyridinium)methyl]ceph-3-em-4-carboxylat

9,88 g 7-Amino-3-[(2,3-cyclopenteno-1-pyridinium)methyl]ceph-3-em-4-carboxylat.Hydrojodid.Monohydrat werden in 100 ml Dichlormethan mit 11,4 ml N,O-Bis(trimethylsilyl)-acetamid silyliert. Die entstandene Lösung wird auf -15° gekühlt und mit 8,4 g 2-(2-Amino-4-thiazolyl)-2-oxothioessigsäure-S-benzthiazol-2-ylester versetzt. Man rührt drei Stunden bei -15° und setzt dann 100 ml Ethanol und 3 ml Triethylamin zu. Der Ansatz wird eine Stunde bei 0° gerührt, der entstandene Niederschlag abfiltriert, gewaschen und getrocknet. Man erhält 11,5 g der Titelverbindung als oranges Pulver.
IR: 3300 NH, 2965 $CH_2$, 1795 c = o (β-Lactam), 1690 c = o, 1635 (Amid I), 1545 (Amid II).

HERSTELLUNG DER AUSGANGSPRODUKTE IV

Beispiel 3 : 2-(2-Aminothiazol-4-yl)-2-oxothioessigsäure-S-benzothiazol-2-ylester:

34,4 g Aminothiazolylglyoxylsäure werden in 500 ml Dichlormethan suspendiert und unter Rühren mit 28 ml Triethylamin versetzt. Man kühlt auf -15° und gibt 64 g Triphenylphosphin und 80 g Bisbenzothiazol-2-yldisulfid zu. Nach zweistündigem Rühren bei -15° wird der orange Niederschlag abfiltriert, mit kaltem Dichlormethan gewaschen und im Vakuum getrocknet. Ausbeute: 57,7 g, d.i. 89,8 %.
IR: 3300 N-H, 1675 Carbonyl (Thioester), 1645 Carbonyl (Amid), 1540 Amidbande.

Beispiel 4 : 2-(2-Aminothiazol-4-yl)-2-oxothioessigsäure-S-benzothiazol-2-ylester:

80 g Bisbenzothiazol-2-yldisulfid und 64 g Triphenylphosphin werden in 500 ml Tetrahydrofuran suspendiert und auf -15° gekühlt. Man gibt 34,4 g Aminothiazolylglyoxylsäure zu und tropft 16,1 ml Pyridin so langsam zu, daß die Innentemperatur nicht über -15° ansteigt. Die orange Suspension wird zwei Stunden bei -15° gerührt. Der Niederschlag wird abfiltriert, mit kaltem Tetrahydrofuran gewaschen und im Vakuum getrocknet. Ausbeute: 62,2 g als Tetrahydrofuran-Solvat. Fp: bei 80° Entweichen von THF, ab 123° (Zers.). THF-Gehalt (gaschromatographisch): 17%

**Ansprüche**

1. Cephalosporinzwischenprodukte der Formel I

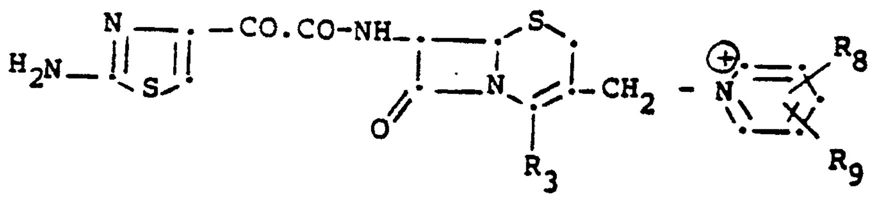

I

worin $R_3$ die Carboxyl-, die Carboxylat- oder eine Carbonsäureestergruppe bedeutet und $R_8$ und $R_9$ gleich oder verschieden sind und jeweils für Wasserstoff, Halogen, Alkyl, Hydroxy, Carboxamido, Alkoxycarbonyl, Amino, Monoalkylamino oder Dialkylamino stehen oder zusammen einen gegebenenfalls substituierten, 5- oder 6-gliedrigen carbocyclischen Ring bedeuten und ihre Salze.

2. Cephalosporinzwischenprodukte der Formel I,
worin $R_3$ die Carboxyl-, die Carboxylat- oder eine Carbalkoxygruppe bedeutet und $R_8$ und $R_9$ gleich oder verschieden sind und jeweils für Wasserstoff, Halogen, Alkyl, Hydroxy, Carboxamido, Alkoxycarbonyl, Amino, Monoalkylamino oder Dialkylamino stehen oder zusammen einen gegebenenfalls substituierten, 5- oder 6-gliedrigen carbocyclischen Ring bedeuten und ihre Salze.

3. Verbindungen der Formel I nach Anspruch 1 oder 2 zur Verwendung als Zwischenprodukt für die Herstellung von Cephalosporinen.